Europäisches Patentamt

European Patent Office

Office européen des brevets

(11) Veröffentlichungsnummer: **0 001 084**
**B1**

(12) **EUROPÄISCHE PATENTSCHRIFT**

(21) Anmeldenummer: 78100768.7

(22) Anmeldetag: 28.08.78

(51) Int. Cl.³: **C 07 C 143/665,**
**C 07 C 97/24,**
**C 07 C 139/00**

(54) Verfahren zur Herstellung von 1-Amino-4-bromanthrachinon-2-sulfonsäure

(30) Priorität: 10.09.77 DE 2740885

(43) Veröffentlichungstag der Anmeldung:
21.03.79 Patentblatt 79/06

(45) Bekanntmachung des Hinweises auf die Patenterteilung:
17.09.80 Patentblatt 80/19

(84) Benannte Vertragsstaaten:
BE CH DE FR GB LU NL

(56) Entgegenhaltungen:
DE - B - 1 135 495
FR - A - 653 159
GB - A - 664 839
US - A - 2 590 247

(73) Patentinhaber: BAYER AG
Zentralbereich Patente, Marken und Lizenzen
D - 5090 Leverkusen 1, Bayerwerk (DE)

(72) Erfinder: Muders, Rolf, Dr.
Ludwig-Aschoff-Strasse 16
D - 5000 Köln 80 (DE)
Leister, Heinrich, Dr.
Treptower Strasse 8
D - 5090 Leverkusen (DE)
Dittmer, Helmut, Dr.
Zedernweg 6
D - 5090 Leverkusen 31 (DE)
Schönhagen, Hubert
Im Schwarzbroich 15
D - 5068 Odenthal (DE)

Verfahren zur Herstellung von 1-Amino-4-bromanthrachinon-2-sulfonsäure

Die Erfindung betrifft ein Verfahren zur Herstellung von 1-Amino-4-bromanthrachinon-2-sulfonsäure aus 1-Amino-anthrachinon.

Die Herstellung von 1-Amino-4-bromanthrachinon-2-sulfonsäure wird üblicherweise in einem 2-Stufen-Verfahren durchgeführt, wobei in Stufe 1 die Sulfonierung des 1-Aminoanthrachinons mit Chlorsulfonsäure in einem mit Wasser nicht mischbaren inerten organischen Lösungsmittel erfolgt. Nach Abtrennung vom organischen Lösungsmittel wird dann in neutraler wäßriger Lösung (vgl. z.B. US—PS 3 428 659) oder in schwefelsaurer Lösung (vgl. z.B. japanische Offenlegungsschrift 49 076 848) bromiert.

Ein anderer zweistufiger Weg besteht darin, 1-Amino-anthrachinon zunächst in SO₃-haltiger Schwefelsäure zu sulfonieren (vgl. DT—PS 263 395 und 484 997), die erhaltene 1-Aminoanthrachinon-2-sulfonsäure zu isolieren und in wäßrigem Medium zu bromieren (vgl. z.B. FIAT 1313 II, S. 214, US—PS 2 413 790, 2 440 760 und 2 503 254, DE—AS 2 303 246).

Gemäß dem in der DE—OS 2 551 767 beschriebenen Verfahren wird während der Sulfonierung noch Borsäure zugesetzt; die Bromierung erfolgt in stark verdünnter wäßriger Schwefelsäure.

Bei den genannten Verfahren wird die Aminosulfonsäure zwischenisoliert bzw. extrahiert, was einen zusätzlichen Verfahrensschritt bedeutet, der das Verfahren technisch aufwendig gestaltet.

Weiterhin wird in Chem. Abstr., Vol. 60, Seite 2874b ein Verfahren zur Herstellung von 1-Amino-4-bromanthrachinon-2-sulfonsäure aus 1-Amino-anthrachinon ohne Zwischenisolierung der 1-Amino-anthrachinon-2-sulfonsäure beschrieben.

Gemäß diesem Verfahren wird jedoch die Sulfonierung in Gegenwart von Borsäure durchgeführt, um unerwünschte Nebenreaktionen, wie die Bohn-Schmidt-Reaktion, zu unterdrücken.

Nach der Sulfonierung wird das Reaktionsgemisch mit Wasser bei Rückflußtemperatur behandelt, um isomere Aminoanthrachinonsulfonsäuren, wie die 4-Sulfonsäure, zu hydrolysieren.

Hierzu wird nach dem Ausführungsbeispiel der CSSR-Patentschrift 105 953 das Sulfiergemisch in einen anderen Behälter überführt, in dem das zur Hydrolyse erforderliche Wasser vorgelegt ist.

Nach der Behandlung des Sulfiergemisches mit Wasser kann das rohe, 1-Aminoanthrachinon-2-sulfonsäure enthaltende Gemisch, das in wäßriger Suspension vorliegt, direkt bromiert werden.

Verfahrenstechnisch handelt es sich bei dem in der obigen Literaturstelle beschriebenen Verfahren um ein "Zweitopf-Verfahren", d.h., die Sulfierung und die Bromierung werden getrennt in zwei verschiedenen Behältern durchgeführt.

Ein solches Verfahren ist aber technisch aufwendig und daher wenig wirtschaftlich.

Außerdem wird in Chem. Abstr., Vol. 60, Seite 670f und in der britischen Patentschrift 664 839, Seite 2, Zeilen 25 bis 37, noch die Bromierung von 1-Aminoanthrachinon-2-sulfonsäure beschrieben.

Gemäß diesen Literaturstellen wird die Bromierung bei Raumtemperatur oder schwach erhöhter Temperatur bzw. bei 15 bis 20°C in Gegenwart von Pyridin und Chlor durchgeführt.

Es wurde nun ein Verfahren zur Herstellung von 1-Amino-4-bromanthrachinon-2-sulfonsäure aus 1-Amino-anthrachinon gefunden, das dadurch gekennzeichnet ist, daß man 1-Aminoanthrachinon mit SO₃-haltiger Schwefelsäure gegebenenfalls unter Zusatz von Alkalimetallsulfaten auf Temperaturen im Bereich von 100 bis 150°C erhitzt und anschließend im gleichen Reaktionsgefäß das Gemisch mit mindestens 0,5 Moläquivalenten Brom, bezogen auf 1 Mol 1-Amino-anthrachinon, bei Temperaturen im Bereich von 60—100°C behandelt.

Bei dem erfindungsgemäßen Verfahren wird die 1-Amino-4-bromanthrachinon-2-sulfonsäure ohne Zwischenisolierung der 1-Aminoanthrachinon-2-sulfonsäure in guten Ausbeuten (über 70% der Theorie) und hoher Reinheit (mindestens 93%ig) erhalten.

Die Sulfonierung nach dem erfindungsgemäßen Verfahren wird in der Regel so durchgeführt, daß man das 1-Amino-anthrachinon in der SO₃-haltigen Schwefelsäure löst, gegebenenfalls Alkalimetallsulfat zugibt, und das Reaktionsgemisch auf die gewünschte Temperatur bringt oder daß man die Komponenten.

Dem Sulfonierungsgemisch können vorteilhafterweise Alkalimetallsulfate, wie Lithiumsulfat, Natriumsulfat, Kaliumsulfat, Rubidiumsulfat, Caeciumsulfat, bevorzugt Natriumund/oder Kaliumsulfat zugesetzt werden. Der Alkalimetallsalzzusatz soll nicht nur den Eintritt einer weiteren Sulfonsäuregruppe, sondern auch die Hydroxylierung in der 4-Position verhindern. Bevorzugt verwendet man wasserfreie Sulfate. Dem Sulfonierungsgemisch werden im allgemeinen 0,1 bis 1,4, bevorzugt 0,4 bis 1,2, Teile Alkalimetallsulfat pro Teil 1-Amino-anthrachinon zugesetzt.

Ohne Zusatz oder in Gegenwart von wenig Alkalimetallsulfat entstehen im allgemeinen höhere Anteile an 1-Aminoanthrachinon-2,4-disulfonsäure als bei Zugabe der dem 1-Aminoanthrachinon etwa gleichen Menge Alkalimetallsulfat.

Bei der Sulfonierung evtl. entstandene 1-Amino-anthrachinon-2,4-disulfonsäure beeinflußt den weiteren Reaktionsverlauf nicht, da die 4-Sulfonsäuregruppe bei allmählich sinkender Schwefelsäurekonzentration wieder eliminiert oder später durch Brom substituiert wird. Diese Eliminierung kann beschleunigt werden, wenn nach der Sulfonierung die Schwefelsäurekonzentration auf ca. 95 bis 100 Gew.-% gesenkt wird.

Der Verlauf der Sulfonierung ist chromatographisch gut zu verfolgen. Im allgemeinen ist die Sulfonierung nach etwa 1 bis 10 Stunden, vorzugsweise nach 2 bis 6 Stunden, beendet.

Die der Sulfonierung sich anschließende Bromierung wird bei niedrigeren Temperaturen, beispielsweise im Bereich von etwa 60 bis 100°C, bevorzugt bei 70 bis 90°C, durchgeführt. Vor der Bromzugabe muß man daher das Reaktionsgemisch auf die gewünschte Bromierungstemperatur abkühlen.

Vorteilhaft für die Bromierung ist der Zusatz eines üblichen Halogenierungskatalysators, wie Jod. Der Zusatz eines solchen Katalysators ist für das erfindungsgemäße Verfahren jedoch nicht unbedingt erforderlich.

Da ein großer Teil des bei der Bromierung entstehenden Bromwasserstoffs im Reaktionsgemisch zu Brom rückoxidiert wird, wird weniger als 1 Moläquivalent Brom pro Mol eingesetztem 1-Amino-anthrachinon verbraucht. Man benötigt mindestens 0,5 Moläquivalente, im allgemeinen 0,6 bis 0,9 Moläquivalente Brom, bevorzugt 0,6 bis 0,7 Moläquivalente Brom, bezogen auf 1 Mol 1-Amino-anthrachinon.

Die Bromierung kann durcklos unter Rückflußkühlung oder unter Druck durchgeführt werden. Üblicherweise arbeitet man drucklos, gelegentlich kann es jedoch vorteilhaft sein, unter Druck bis zu etwa 6 bar zu arbeiten.

Der Verlauf der Bromierung läßt sich chromatographisch ebenfalls gut verfolgen. Im allgemeinen ist die Bromierung nach etwa 4 bis 16 Stunden beendet.

Das Reaktionsgemisch läßt sich vorteilhafterweise so aufarbeiten, daß man nach Entfernen von überschüssigem Brom durch Mischen mit Wasser oder verdünnter Schwefelsäure die Schwefelsäurekonzentration des Reaktionsgemisches auf etwa 60 bis 85 Gew.-%, vorzugsweise 65 bis 80 Gew.-%, einstellt. Es empfiehlt sich, den Zusatz zunächst mit einer Schwefelsäure etwas höherer Konzentration (beispielsweise 70%ig) zu beginnen. Hierbei scheidet sich die 1-Amino-4-bromanthrachinon-2-sulfonsäure als gut kristallisiertes Sulfat ab. Die Fällungstemperatur liegt bei etwa 50 bis 60°C und kann zur Verbesserung der Filtrationsform um weitere 30 bis 40°C angehoben werden. Das ausgefällte Sulfat wird durch Filtration isoliert und von der anhaftenden Mutterlauge durch Waschen mit ca. 60 bis 85%iger Schwefelsäure befreit.

Durch Anrühren in Wasser wird das so gewonnene Sulfat hydrolisiert, wobei durch Zugabe von Alkalisalzen, wie Natriumchlorid, Kaliumchlorid, Natriumsulfat und/oder Kaliumsulfat, oder von Alkalien, wie Natron- oder Kalilauge oder Natriumcarbonat, die 1-Amino-4-anthrachinon-2-sulfonsäure als Salz ausgefällt wird.

Diese Art der Aufarbeitung liefert günstige Voraussetzungen für eine Aufbereitung der anfallenden Schwefelsäure, deren Konzentration über 60 Gew.-% liegt, durch bekannte Methoden der Aufkonzentrierung.

Eine andere Art der Aufarbeitung des Reaktionsgemisches nach der Bromierung besteht darin, daß dieses unter Rühren in vorgelegtes Wasser oder Eiswasser gegeben wird, dem gegebenenfalls Natriumchlorid, Kaliumchlorid, Natriumsulfat und/oder Kaliumsulfat zugesetzt wird. Die ausgefällte 1-Amino-4-bromanthrachinon-2-sulfonsäure wird durch Filtration aus der sauren Suspension isoliert. Dem Filtrationsgut haften je nach Reaktionsverlauf bis zu insgesamt 5% Verunreinigung an, bestehend aus geringen Mengen von Nebenprodukten wie 1-Amino-2- bzw. -4-bromanthrachinon, 1-Amino-2,4-dibromanthrachinon bzw. 1-Amino-anthrachinon-2-sulfonsäure.

Die Abtrennung der wasserunlöslichen Anteile erfolgt durch eine Klärfiltration des in Wasser gelösten Reaktionsproduktes in saurem und/oder alkalischem Bereich, vorteilhaft in Gegenwart von Filtrationshilfsmitteln, wie Aktivkohle oder Kieselgur.

Aus dem Filtrat kann die 1-Amino-4-bromanthrachinon-2-sulfonsäure durch Aussalzen, beispielsweise mit Natriumchlorid, Kaliumchlorid, Natriumsulfat und/oder Kaliumsulfat, in Form ihres Alkalisalzes ausgefällt werden. Man isoliert die 1-Amino-4-bromanthrachinon-2-sulfonsäure durch Filtration und wäscht mit verdünnter Salzlösung nach und trocknet das so erhaltene Produkt.

Der Vorteil des erfindungsgemäßen Verfahrens liegt in der einstufigen Verfahrensweise. Dadurch gestaltet sich das erfindungsgemäße Verfahren besonders wirtschaftlich. Die physikalische Beschaffenheit und die Qualität des eingesetzten 1-Aminoanthrachinons sind ohne Einfluß auf den Reaktionsablauf. 1-Amino-4-bromanthrachinon-2-sulfonsäure ist ein wichtiges Zwischenprodukt für zahlreiche wertvolle Farbstoffe (vgl. Ullmann Encyklopädie der technischen Chemie, 4. Auflage, Band 7, S. 639—640).

Da bei dem erfindungsgemäßen Verfahren weder das Sulfiergemisch vor der Bromierung mit überschüssigem Wasser zur Beseitigung von unerwünschten isomeren Sulfonsäuren behandelt noch die Bromierung bei teifen Temperaturen, wie bisher üblich, in Gegenwart von Pyridin als Säurefänger und Chlor als Oxidationsmittel durchgeführt wird (vgl. z.B. die eingangs erwähnte britische Patentschrift 664 839 und Chem. Abstr., Vol. 60, Seite 670f), ist es außerordentlich überraschend, daß nach dem erfindungsgemäßen Verfahren solch gute Ausbeuten und Reinheiten an 1-Amino-4-brom-anthrachinon-2-sulfonsäure erhalten werden.

Es war vielmehr zu erwarten, daß unter den erfindungsgemäßen Reaktionsbedingungen, die im Vergleich zu den literaturbekannten Bedingungen als drastisch angesehen werden können (höhere Reaktionstemperatur, höhere Schwefelsäurekonzentration, Fehlen von Borsäure und Pyridin), Nebenreaktionen stattfinden würden, was eine Beeinträchtigung der Ausbeuten und Reinheiten zur Folge hätte.

Die folgenden Beispiele sollen das erfindungsgemäße Verfahren näher erläutern, ohne es jedoch auf diese Beispiele einzuschränken.

### Beispiel 1

In ein Gemisch von 200 ml 20%igem Oleum und 100 g wasserfreiem Natriumsulfat trägt man unter Rühren 150 g 96%iges 1-Amino-anthrachinon ein und erhitzt innerhalb 1 Stunde auf 130°C. Man hält diese Temperatur 2 Stunden lang, setzt 120 ml 20%iges Oleum und 50 g wasserfreies Natriumsulfat zu und verrührt weitere 3 Stunden bei 130°C. Die Sulfonierung ist dann praktisch beendet. Zur Bromierung kühlt man auf 80°C ab, fügt 0,2 g Jod und ca. 0,5 ml eines Entschäumers hinzu und läßt bei 80°C innerhalb von 9 Stunden 21 ml Brom portionsweise zulaufen. Der Gehalt an 1-Amino-anthrachinon-2-sulfonsäure in einer aufgearbeiteten Probe liegt danach unter 1%.

Zur Aufarbeitung entfernt man zunächst überschüssiges Brom durch kurzes Evakuieren und läßt dann innerhalb 1 Stunde 140 ml Wasser zutropfen, wobei die Temperatur von 80°C auf 110°C ansteigt. Man saugt das Bromaminsäuresulfat bei 50 bis 60°C ab und wäscht mit 300 ml 60%iger Schwefelsäure nach. Mutterlauge und Wäsche werden vereinigt und ergeben 855 g einer 65%iger Schwefelsäure, die nach bekannten Verfahren aufkonzentriert werden kann.

Den Nutschkuchen (668 g) rührt man in 1800 ml Wasser an, stellt durch Zugabe von 308 ml 50%iger Natronlauge einen ph-Wert von 8 ein und erhitzt 1 Stunde auf 90°C. Dann kühlt man auf 60°C ab, saugt das Natriumsalz der 1-Amino-4-bromanthrachinon-2-sulfonsäure ab, wäscht es mit 1200 ml einer 1,5%iger Natriumsulfatlösung und trocknet es. Man erhält 234,6 g mit folgender Analyse:

87,5% 1 - Amino - 4 - bromanthrachinon - 2 - sulfonsäure
0,5% 1-Amino-anthrachinon-2-sulfonsäure
0,4% unsulfonierte Bestandteile
7,1% Wasser

Die Ausbeute an 1-Amino-4-bromanthrachinon-2-sulfonsäure beträgt demnach 83,2% der Theorie, bezogen auf 1-Amino-anthrachinon.

Bezogen auf wasserfreie Substanz liegt die Reinheit der 1-Amino-4-bromanthrachinon-2-sulfonsäure bei 94,2%.

### Beispiel 2

In 260 ml 100%ige Schwefelsäure trägt man unter Rühren 146 g wasserfreies Natriumsulfat und 150 g 1-Aminoanthrachinon ein und löst bei 80 bis 85°C. Zur Sulfonierung erhitzt man auf 120°C und fügt dann gleichmäßig innerhalb 3 Stunden 94 ml 65%iges Oleum zu. Nach weiterem zweistündigem Rühren ist die Sulfonierung beendet, was chromatographisch nach üblicher Methode verfolgt werden kann.

Zur Bromierung kühlt man auf 50°C ab, verdünnt mit 18 ml 78%iger Schwefelsäure und setzt 0,8 g Jod zusammen mit 73 g Brom zu. Unter Rückfluß erhitzt man das Reaktionsgemisch 6 Stunden lang auf 60°C, stellt dann durch Zugabe verdünnter Schwefelsäure die Schwefelsäurekonzentration des reaktionsgemisches auf 95% und bromiert weitere 12 Stunden bei 60 bis 70°C.

Zur weiteren Aufarbeitung verfährt man wie in Beispiel 1 beschrieben, durch Fällung und Isolierung des Sulfates aus schwefelsaurer Lösung mit anschließender Hydrolyse, wobei man die 1-Amino-4-bromanthrachinon-2-sulfonsäure in gleicher Qualität und Ausbeute wie in Beispiel 1 erhält.

### Beispiel 3

Die Sulfonierung und Bromierung werden wie in Beispiel 1 bzw. 2 beschrieben durchgeführt.

Zur Aufarbeitung entfernt man überschüssiges Brom durch kurzzeitiges Evakuieren aus der Reaktionsschmelze und stellt durch langsame Zugabe von 30%iger Schwefelsäure die Konzentration der Schwefelsäure im Reaktionsgemisch auf ca. 70%. Dabei fällt das Sulfat der 1-Amino-4-bromanthrachinon-2-sulfonsäure aus, welches abfiltriert und mit 300 ml 60%iger Schwefelsäure gewaschen wird. Die gesammelten Filtrationsabläufe enthalten 65 bis 68% Schwefelsäure, die nach üblicher Methode aufkonzentriert werden kann.

Das Filtrationsgut rührt man in 300 ml Wasser an und filtriert erneut. Das anfallende Filtrat mit einem Gehalt von 30% Schwefelsäure dient nach Art eines Recyclingprozesses zum Verdünnen der Reaktionsschmelze wie oben beschreiben.

Zur Neutralisation der isolierten 1-Amino-4-bromanthrachinon-2-sulfonsäure wird in 2000 ml Wasser angerührt und die Suspension mit 50%iger Natronlauge auf pH 9 gestellt. Man erhitzt unter Rühren 15 Minuten auf 90°C, kühlt auf 50 bis 60°C, filtriert und wäscht mit einer 1,5%iger wäßrigen Natriumsulfatlösung.

Ausbeute und Qualität des so erhaltenen Produktes sind wie unter Beispiel 1 beschrieben.

### Beispiel 4

Die Sulfonierung und Bromierung werden wie unter Beispiel 1 bzw. 2 durchgeführt und das Reaktionsgemisch wie folgt aufgearbeitet:

Man kühlt auf 20°C und trägt das Reaktionsgemisch unter Rühren in 1200 g Eiswasser ein, in dem 27 g Natriumchlorid gelöst sind. Die so ausgefällte 1-Amino-4-bromanthrachinon-2-sulfonsäure wird abfiltriert und in 3000 ml Wasser angerührt. Durch Zusatz von Natronlauge 50%ig wird der pH-Wert auf 8 bis 9 gestellt. Beim Erhitzen auf 95 bis 100°C tritt Lösung ein. Man fügt 12 g einer Mischung aus Aktiv-Kohle und Kieselgur zu, hält 1/2 Stunde weiter auf Temperatur und filtriert heiß. Den Filtrationsrückstand wäscht man mit 400 ml heißem Wasser nach.

Aus den gesammelten Filtraten wird das Salz der 1-Amino-4-bromanthrachinon-2-sulfonsäure mit 50 g Natriumsulfat bei 90 bis 95°C ausgefällt. Vor der Filtration kühlt man auf 40°C ab.

Die 1-Amino-4-bromanthrachinon-2-sulfonsäure fällt mit einer Reinheit von 93 bis 94%, bezogen auf wasserfreie Substanz, in über 70%iger Ausbeute an.

### Beispiel 5

In 150 ml 20%iges Oleum werden 20 g wasserfreies Natriumsulfat gelöst und unterhalb 60°C 150 g 1-Amino-anthrachinon eingetragen. Das Reaktionsgemisch wird 3 Std. auf 110°C erhitzt, auf 80°C abgekühlt und nach Zusatz von 55 ml 20%igem Oleum weitere 3 Std. auf 110°C erhitzt. Anschließend fügt man ca. 0,1—0,2 g Jod und bei 80°C langsam 24 ml Brom zu. Nach ca. 16 Stunden ist die Bromierung durch Erwärmen des Reaktionsgemisches unter Rückfluß auf 80°C beendet. Das überschüssige Brom wird durch Evakuieren oder Ausblasen sorgfältig entfernt und durch langsame Verdünnung mit 135 ml 30%iger Schwefelsäure das Sulfat der 1-Amino-4-bromanthrachinon-2-sulfonsäure ausgefällt. Man filtriert, wäscht mit 300 ml 70%iger Schwefelsäure und danach mit 200 ml 30%iger Schwefelsäure. Zur Hydrolyse wird das Reaktionsprodukt in 2000 ml Wasser angerührt und mit 25%iger Natronlauge bei 95°C auf pH = 1 gestellt. Dabei tritt Lösung ein. Nach weiterem Zusatz von Natronlauge bis pH = 7 kristallisiert das Natriumsalz der 1-Amino-4-bromanthrachinon-2-sulfonsäure aus. Man stellt mit wenig Soda auf pH 8—9, filtriert bei 40—60°C und wäscht mit einer verdünnten Natriumsulfatlösung. Ausbeute 236 g Natriumsalz mit einem Reingehalt von 86,7%.

### Beispiel 6

In 150 ml 20%iges Oleum werden unter Kühlung (Temperatur <60°C) 150 g 1-Amino-anthrachinon eingetragen und das Reaktionsgemisch in ca. 1 Stunde auf 110°C erhitzt. Diese Temperatur wird ca. 2 Stunden lang gehalten, dann bei 90°C 55 ml 20%iges Oleum langsam zugesetzt und die Sulfonierung bei 110°C fortgeführt. Nach Beendigung der Reaktion (Dauer mindestens 2 Stunden) wird das Reaktionsgemisch wie unter Beispiel 5 beschrieben, bromiert und aufgearbeitet.

Ausbeute 226 g Natriumsalz mit einem Reingehalt von 86%.

### Beispiel 7

Die Sulfonierung wird wie in Beispiel 5 beschrieben durchgeführt. Zur Bromierung setzt man dem Reaktionsgemisch 0,15 g Jod und 62,5 g Brom zu und erhitzt in einem geschlossenen Reaktionsgefäß unter Rühren 16 Stunden lang auf 80°C. Dabei steigt der Druck auf 3,5—4 bar. Nach Abkühlung auf 60°C wird das Reaktionsgefäß belüftet, um danach überschüssiges Brom und Bromwasserstoff durch Ausblasen bzw. Evakuieren sorgfältig aus dem Reaktionsgemisch zu entfernen.

Zur aufarbeitung werden langsam unter Rühren 108 ml 70%ige Schwefelsäure und danach 140 ml 30%ige Schwefelsäure zugefügt, wobei das 1-Amino-4-bromanthrachinon-2-sulfonsäure in Form ihrer Sulfates ausfällt. Man filtriert, wäscht mit 375 ml 70%iger Schwefelsäure und mit 234 ml 30%iger Schwefelsäure.

Die weitere Aufarbeitung durch Hydrolyse erfolgt wie unter Beispiel 5 beschrieben, wobei man wie dort die 1-Amino-4-bromanthrachinon-2-sulfonsäure in gleich guter Qualität und Ausbeute erhält.

## Patentansprüche

1. Verfahren zur Herstellung von 1-Amino-4-bromanthrachinon-2-sulfonsäure aus 1-Amino-anthrachinon, dadurch gekennzeichnet, daß man 1-Amino-anthrachinon mit $SO_3$-haltiger Schwefelsäure gegebenenfalls unter Zusatz von Alkalimetallsulfaten auf Temperaturen im Bereich von 100 bis 150°C erhitzt und anschließend im gleichen Reaktionsgefäß das Gemisch mit mindestens 0,5 Moläquivalenten Brom, bezogen auf 1 Mol 1-Amino-anthrachinon, bei Temperaturen im Bereich von 60 bis 100°C behandelt.

2. Verfahren nach Anspruch, dadurch gekennzeichnet, daß der $SO_3$-Gehalt der Schwefelsäure 4 bis 40 Gew.-%, bezogen auf 100 Gew.-%ige Schwefelsäure, beträgt.

3. Verfahren nach Ansprüchen 1 und 2, dadurch gekennzeichnet, daß man die $SO_3$-haltige Schwefelsäure in der 2- bis 8-fachen Gewichtsmenge, bezogen auf eingesetztes 1-Amino-anthrachinon, zusetzt.

4. Verfahren nach Ansprüchen 1 bis 3, dadurch gekennzeichnet, daß man als Alkalimetallsulfate Natrium- und/oder Kaliumsulfat verwendet.

5. Verfahren nach Ansprüchen 1 bis 4, dadurch gekennzeichnet, daß man 0,1 bis 1,4 Teile Alkalimetallsulfat pro Teil 1-Amino-anthrachinon verwendet.

6. Verfahren nach Ansprüchen 1 bis 5, dadurch gekennzeichnet, daß man die Bromierung mit 0,6 bis 0,9 Moläquivalenten Brom, bezogen auf 1 Mol 1-Amino-anthrachinon, durchführt.

7. Verfahren nach Ansprüchen 1 bis 6, dadurch gekennzeichnet, daß man zur Isolierung der 1-Amino-4-bromanthrachinon-2-sulfonsäure die Schwefelsäurekonzentration im Reaktionsgemisch durch Mischen des Reaktionsgemisches mit Wasser oder verdünnter Schwefelsäure auf 60 bis 85 Gew.-% einstellt und das sich abscheidende Sulfat der 1-Amino-4-bromanthrachinon-2-sulfonsäure filtriert und durch Einwirkung von Wasser und/oder Alkalien in 1-Amino-4-bromanthrachinon-2-sulfonsäure oder deren Alkalimetallsalze überführt.

## Claims

1. Process for the preparation of 1-Amino-4-bromo-anthraquinone-2-sulphonic acid from 1-amino-anthraquinone, characterised in that 1-amino-anthraquinone is heated to temperatures in the range from 100 to 150°C with $SO_3$-containing sulphuric acid, alkali metal sulphates being added if necessary and the mixture is then treated in the same reaction vessel with at least 0.5 molar equivalent of bromine, relative to 1 mol of 1-amino-anthraquinone, at temperatures in the range from 60—100°C.

2. Process according to Claim 1, characterized in that the $SO_3$ content of the sulphuric acid is 4 to 40% by weight, relative to 100% strength by weight sulphuric acid.

3. Process according to Claims 1 and 2, characterised in that the $SO_3$-containing sulphuric acid is added in 2 to 8 times the amount by weight, relative to 1-aminoanthraquinone employed.

4. Process according to Claims 1 to 3, characterised in that sodium sulphate and/or potassium sulphate are used as the alkali metal sulphates.

5. Process according to Claims 1 to 4, characterised in that 0.1 to 1.4 parts of alkali metal sulphate are used per part of 1-aminoanthraquinone.

6. Process according to Claims 1 to 5, characterised in that the bromination is carried out with 0.6 to 0.9 molar equivalent of bromine, relative to 1 mol of 1-amino-anthraquinone.

7. Process according to Claims 1 to 6, characterised in that in order to isolate the 1 - amino - 4 - bromo - anthraquinone - 2 - sulphonic acid, the sulphuric acid concentration in the reaction mixture is adjusted to 60 to 85% by weight by mixing the reaction mixture with water or dilute sulphuric acid, and the sulphate of 1 - amino - 4 - bromo - anthraquinone - 2 - sulphonic acid which separates is filtered off and converted into 1 - amino - 4 - bromo-anthraquinone - 2 - sulphonic acid and/or its alkali metal salts by the action of water and/or water which contains alkali metal salts.

## Revendications

1. Procédé de préparation d'acide 1 - amino - 4 - bromanthraquinone - 2 - sulfonique à partir de 1 - amino - anthraquinone, caractérisé en ce qu'on chauffe de la 1-amino-anthraquinone avec de l'acide sulfurique contenant du $SO_3$ à des températures allant de 100 à 150°C en ajoutant éventuellement des sulfates de métaux alcalins puis, dans le même récipient réactionnel, on traite le mélange avec au moins 0,5 équivalent molaire de brome, calculé sur 1 mole de 1-amino-anthraquinone, à des températures allant de 60 à 100°C.

2. Procédé suivant la revendication 1, caractérisé en ce que la teneur en $SO_3$ de l'acide sulfurique est de 4 à 40% en poids, calculés sur l'acide sulfurique à 100% en poids.

3. Procédé suivant l'une quelconque des revendications 1 et 2, caractérisé en ce qu'on ajoute l'acide sulfurique contenant du $SO_3$ en une quantité pondérale égale à 2—8 fois celle de la 1-amino-anthraquinone utilisée.

4. Procédé suivant l'une quelconque des revendications 1 à 3, caractérisé en ce que, comme sulfates de métaux alcalins, on utilise le sulfate de sodium et/ou le sulfate de potassium.

5. Procédé suivant l'une quelconque des revendications 1 à 4, caractérisé en ce qu'on utilise 0,1 à 1,4 partie d'un sulfate de métal alcalin par partie de 1-amino-anthraquinone.

6. Procédé suivant l'une quelconque des revendications 1 à 5, caractérisé en ce qu'on effectue la bromation avec 0,6 à 0,9 équivalent molaire de brome, calculé sur 1 mole de 1-amino-anthraquinone.

7. Procédé suivant l'une quelconque des revendications 1 à 6, caractérisé en ce que, pour l'isolation de l'acide 1 - amino - 4 - bromanthraquinone - 2 - sulfonique, on règle la concentration d'acide sulfurique dans le mélange réactionnel à 60—85% en poids en mélangeant le mélange réactionnel avec de l'eau ou de l'acide sulfurique dilué, puis on filtre le sulfate de l'acide 1 - amino - 4 - bromanthraquinone - 2 - sulfonique qui se sépare et on le transforme en acide 1 - amino - 4 - bromanthraquinone - 2 - sulfonique ou ses sels de métaux alcalins sous l'action de l'eau et/ou d'alcalis.